# EUROPÄISCHE PATENTSCHRIFT

(11) **EP 2 819 732 B1**
(45) Veröffentlichungstag und Bekanntmachung des Hinweises auf die Patenterteilung: **03.05.2017**
(21) Anmeldenummer: 12773280.8
(22) Anmeldetag: 27.09.2012
(51) Int. Cl.: A61M 16/08, A61M 16/16, A61M 16/00, A61M 16/10

(54) **BEATMUNGSSCHLAUCH**
RESPIRATORY TUBE
TUYAU DE VENTILATION

(30) Priorität: 02.03.2012 DE 102012101795
(43) Veröffentlichungstag der Anmeldung: 07.01.2015
(73) Patentinhaber: Hamilton Medical AG, 7402 Bonaduz (CH)
(72) Erfinder: BÜCHI, Rudolf, 7000 Chur (CH)
(74) Vertreter: Caspary, Karsten
(86) Internationale Anmeldenummer: PCT/EP2012/069147
(87) Internationale Veröffentlichungsnummer: WO 2013/127474

(56) Entgegenhaltungen:
- EP-A2- 0 672 430
- EP-A2- 1 222 940
- EP-A2- 2 229 973
- WO-A1-2010/073161
- DE-A1- 4 438 216
- GB-A- 1 294 307
- GB-A- 2 277 689
- US-A- 4 727 871
- US-B1- 7 497 215

## Beschreibung

Die vorliegende Erfindung betrifft einen Beatmungsschlauch für das Durchleiten eines Gasgemisches in einem Beatmungsgerät zum Beatmen von Patienten sowie ein Beatmungsschlauchsystem.

Bei der maschinellen Beatmung von Patienten, die beispielsweise auf einer Intensivstation liegen, wird der zu beatmende Patient mit Hilfe eines Beatmungsschlauchsystems pneumatisch mit dem Beatmungsgerät verbunden. Da das Atemgas, das dem Patienten zugeführt wird, hinsichtlich Temperatur und Feuchtigkeit den physiologischen Erfordernissen des Patienten angepasst werden muss, wird ein Atemluftbefeuchter in dem Einatem- oder Inspirationsschlauch angeordnet, der das Atemgas anwärmt und anfeuchtet. Der Atemluftbefeuchter weist einen in üblicher Weise mit destilliertem Wasser gefüllten Flüssigkeitsbehälter auf, durch den das Einatemgas geleitet und mit Feuchtigkeit angereichert wird.

Um eine Kondensation von Feuchtigkeit innerhalb des Beatmungsschlauchsystems zu verhindern, sind der Inspirationsschlauch und der Exspirations- oder Ausatemschlauch mit einer elektrischen Schlauchheizung versehen, die in Betrieb das durchströmende Ein- oder Ausatemgas erwärmt. Dabei wird beispielsweise eine sich integral innerhalb des Inspirations- oder Exspirationsschlauchs erstreckende Heizdrahtschleife verwendet oder der Inspirations- oder Exspirationsschlauch ist jeweils mit einer Heizdrahtwendel umwickelt.

Derartige Beatmungsschlauchsysteme sind zum Beispiel aus der DE 10 2008 039 137 B3, der DE 10 2007 003 455 A1 oder der DE 44 41 380 A1 bekannt.

Im exspiratorischen Teil des Beatmungsschlauchsystems können insbesondere bei Patienten mit ansteckenden Krankheiten Filter eingesetzt werden, um zu verhindern, dass mit dem Ausatemgas Krankheitserreger in die Umgebung gelangen, wodurch sich weitere Patienten oder Betreuungspersonal anstecken könnten. Ein solcher Filter muss so ausgelegt sein, dass sich darin kein Kondensat bildet, weil dies den Filter verstopfen und dadurch die Filterfunktion beeinträchtigen kann. Im schlimmsten Fall kann auch die Beatmungsfunktion fehlerhaft sein, was zu negativen Folgen für den Patienten führen kann. Üblicherweise wird dies mit zusätzlichen Heizvorrichtungen und/oder mit Wasserfallen erreicht, wie beispielsweise in der US 4,727,871 offenbart. Die allgemeine Verwendung eines Filterelements bei Beatmungsgeräten ist beispielsweise in der US 3,556,097 offenbart.

Die GB 1 294 307 A beschreibt einen beheizbaren Anästhesiefilter, der auf der Inspirationsseite eines Beatmungsgeräts eingesetzt wird. Dabei wird über einen hermetisch abgedichteten Behälter, der einen Filter enthält, eine Hülle aufgesetzt, in die eine Heizung integriert ist. Die Verbindung des Behälters zu den Inspirationsschläuchen erfolgt über Anschlussstutzen, die jeweils am Anfang und Ende des Behälters angeordnet sind.

Die EP 1 222 940 A2 offenbart eine Anästhesiefilteranordnung mit einem einteiligen Gehäuse mit einer oberen und unteren Öffnung für des Atemgasdurchfluss, wobei im Inneren des Gehäuses ein Filterelement quer zur Atemgasflussrichtung angeordnet ist. Innerhalb des Filterelementvolumens ist ein resistives Heizelement angeordnet, um das Filterelement zu erwärmen.

Die GB 2 277 689 A beschreibt einen Filter bzw. eine Wärme- und Feuchtigkeitsaustauschvorrichtung (HME) für ein Beatmungsgerät. Dabei ist ein einteiliges Gehäuse, das in seinem Inneren einen Filter über den gesamten Querschnitt aufweist, von einer als Hülse ausgebildeten Heizeinrichtung umgeben. Die Wärme überträgt sich von Widerstandsdrähten in der Hülse auf das Gehäuse und von dort auf den im Inneren angeordneten Filter.

Die EP 1 222 940 A2 und die GB 2 277 689 A offenbaren einen Beatmungsschlauch mit den Merkmalen des Oberbegriffs des Anspruchs 1.

Nachteilig an den oben erwähnten Beatmungsschlauchsystemen des Standes der Technik ist, dass die Beheizung von Filtern zusätzliche Bauelemente wie Kabel, Anschlüsse usw. erforderlich macht, was aufgrund der Vielzahl von zu verbindenden Kabeln und Schläuchen zu Zeitverlust und möglicher Verwirrung beim Bedienpersonal führen kann, störend in der Patientenumgebung und anfällig für Beschädigungen ist. Die alternative Verwendung von nicht beheizten Filtern birgt die Gefahren des Verstopfens aufgrund von Kondensatbildung wie oben beschrieben.

Es ist daher die Aufgabe der vorliegenden Erfindung, einen Beatmungsschlauch bereit zu stellen, der die Filterung des Ausatemgases wirksam, zuverlässig und dauerhaft ermöglicht, die Kondensation von Wasser im Filter verhindert und die Anzahl zusätzlichen Elementen wie Schläuchen, elektrischen Leitungen sowie deren Anschlüsse und dergleichen minimiert.

Diese Aufgabe wird durch die Merkmale des Anspruchs 1 gelöst. Vorteilhafte Ausgestaltungen und Ausführungsformen sind Gegenstand der Unteransprüche.

Erfindungsgemäß wird ein Beatmungsschlauch für das Durchleiten eines Gasgemisches in einem Beatmungssystem bereitgestellt mit den Merkmalen des Anspruchs 1 Durch die Wärmeübertragung z. B. durch Konvention oder Wärmeleitung von Koppelteil auf das Filterelement wird sichergestellt, dass sich bei der Filterung des Gasgemisches kein Kondensat bildet, das das Filterelement bzw. den Filter verstopfen könnte. Weiterhin ist der Beatmungsschlauch einfach und platzsparend aufgebaut und kommt außer dem Anschlussteil ohne zusätzliche Elemente aus.

Mit besonderem Vorteil sind das Filterelement und das Anschlussteil integral ausgebildet. Dadurch ergibt sich eine weitere Vereinfachung des Beatmungsschlauchs, weil weniger Bauelemente erforderlich sind.

Bevorzugt weisen der Schlauchabschnitt, das Koppelteil und das Anschlussteil einen kreisförmigen Querschnitt auf. Damit können handelsübliche, zylinderförmige Filterelemente verwendet werden. Die Handhabung wie das Zusammensetzen oder gegebenenfalls das Zerlegen der einzelnen Elemente ist ebenfalls vereinfacht.

Mit weiterem Vorteil ist die Verbindung zwischen Koppelteil und Anschlussteil lösbar als Schraub-, Steck- oder Bajonettverschluss ausgebildet. Derartige Verschlüsse sind leicht zu öffnen und zu schließen, was die Handhabung erleichtert, und deren Anwendung ist im Klinikbereich nicht ungewöhnlich.

Damit der Beatmungsschlauch bei aktuellen Beatmungsgeräten in Kliniken eingesetzt werden kann, ist er vorteilhafter Weise als medizinischer Einmal-/Wegwerfartikel ausgebildet.

Bevorzugt weist der Schlauchabschnitt eine zweite elektrische Heizvorrichtung auf, die mit der ersten elektrischen Heizvorrichtung verbindbar ist. Mit weiterem Vorteil sind die erste und/oder die zweite elektrische Heizvorrichtung zumindest abschnittsweise als Heizwendel ausgebildet. Dabei ist die zweite elektrische Heizvorrichtung bevorzugt in die Wand des Schlauchabschnitts integriert. Eine Heizwendel stellt eine einfache Konstruktion dar und sorgt für eine wirksame Beheizung des Beatmungsschlauchs und damit für eine Wärmeübertragung auf das Gasgemisch im Beatmungsschlauch. Die Verbindung zwischen der ersten und zweiten elektrischen Heizvorrichtung ist durch einfache Steckverbinder möglich, die preiswert und robust sind.

Weiterhin sind vorteilhafter Weise der Schlauchabschnitt und das Koppelteil integral ausgebildet. Auch dadurch ergibt sich eine besonders einfache Konstruktion, beispielsweise durch Koextrusion der Bauelemente. Je weniger Elemente der Beatmungsschlauch bzw. das gesamte Beatmungsschlauchsystem aufweisen, desto geringer ist der Aufwand für das Bedienpersonal beim Zusammensetzen und dergleichen.

Durch die Anordnung von Koppelteil und Filterelement wird eine maximale Wärmeübertragung von der ersten elektrischen Heizvorrichtung auf das Filterelement ermöglicht. Die Wärmeübertragung erfolgt dabei beispielsweise durch Konvektion oder auch durch Wärmeleitung. Es dabei darauf zu achten, dass die Filterfunktion des Filterelements nicht beeinträchtigt wird, d. h. das Gasgemisch muss in der gewünschten Strömungsgeschwindigkeit durch den Filter fließen können, um die Beatmungsfunktion nicht zu beeinträchtigen.

Das Filterelement ist dabei bevorzugt zylinderförmig ausgebildet ist, wobei der Abstand zwischen äußerer Mantelfläche des Filterelements und innerer Mantelfläche des Koppelteils ungefähr 0,1 mm bis etwa 8 mm beträgt. Derartige zylinderförmige Filter werden industriell hergestellt und sind kommerziell erhältlich. Ein Beispiel für die Konstruktion eines solchen Filters ohne Beheizung vom Prinzip her ist in den Fig. 11 bis 13 der US 3,556,097 dargestellt. Hierbei wird ein ringförmiger Filter axial innerhalb eines Gehäuses angeordnet, und das Atemgas strömt von der äußeren Mantelfläche des Rings durch das Filtermaterial hindurch zur inneren Mantelfläche und von dort durch die Ausströmöffnung des Gehäuses. Es versteht sich jedoch, dass bei der vorliegenden Erfindung auch andere Konstruktionen von Filterelementen mit Anschluss- und Koppelteil verwendet werden können. Beispielsweise ist ein im Wesentlichen kugelförmiges Filterelement denkbar, das einen Hohlraum aufweist, in den das Gasgemisch von der äußeren Oberfläche durch das Filtermaterial hindurch strömt.

Mit weiterem Vorteil weist der Beatmungsschlauch einen Sensor zur Erfassung von Parametern der Verbindung zwischen Koppelteil und Anschlussteil auf. Damit lässt sich über eine Benutzerschnittstelle, beispielsweise eines Atemluftbefeuchter oder auf eines Beatmungsgerätes, eine Anzeige aktivieren, die die ordnungsgemäße Verbindung zwischen Koppelteil und Anschlussteil anzeigt. Damit kann die Funktion des Filterelements besser überwacht, fehlerhafte Montage bzw. Fehlfunktionen können schneller erkannt werden.

Mit besonderem Vorteil weist das Anschlussteil eine elektrische Heizvorrichtung auf, die elektrisch mit der Heizvorrichtung des Koppelteils verbindbar ist. Dadurch kann die Heizleistung für das Filterelement verstärkt werden, wobei eine einfache elektrische Anbindung beispielsweise über die Verschlusselemente von Koppelteil und Anschlussteil erfolgen kann. Damit kann auch die oben genannte Funktion eines Sensors realisiert sein, wobei beispielsweise das Vorliegen einer wirksamen elektrischen Verbindung zwischen Koppelteil und Anschlussteil die ordnungsgemäße Verbindung angibt. Alternativ kann die Heizvorrichtung des Anschlussteils und gegebenenfalls des Koppelteils auch über ein Verbindungselement gespeist sein, das direkt mit dem Anschlussteil verbunden ist.

Weiterhin erfindungsgemäß ist ein Beatmungsschlauchsystem mit einem Inspirationsschlauch und einem Exspirationsschlauch, der als Beatmungsschlauch wie oben erwähnt ausgebildet ist.

Die Erfindung soll nachfolgend anhand von Ausführungsbeispielen unter Bezugnahme auf die beigefügten Figuren erläutert werden, in denen:
- Fig. 1: eine schematische Darstellung einer bevorzugten Ausführungsform des Beatmungsschlauchsystems der vorliegenden Erfindung zeigt; und
- Fig. 2: eine perspektivische Ansicht einer bevorzugten Ausführungsform des Beatmungsschlauchs gemäß der vorliegenden Erfindung zeigt.

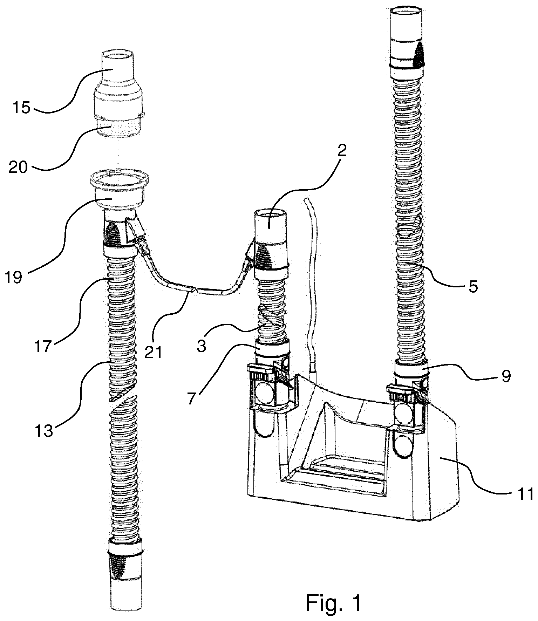

Fig. 1 zeigt eine perspektivische Darstellung des erfindungsgemäßen Beatmungsschlauchssystems. Ein (in Fig. 1 nicht dargestelltes) Beatmungsgerät ist über ein erstes Anschlusselement mit einem ersten Inspirationsschlauch 3 verbunden. Dieser erste Inspirationsschlauch 3 sowie ein zweiter Inspirationsschlauch 5 sind über entsprechende Anschlussstücke 7, 9 mit einem Flüssigkeitsbehälter 11 eines (in Fig. 1 nicht dargestellten) Atemluftbefeuchters verbunden. Das freie Ende des zweiten Inspirationsschlauchs 5 ist mit einem (ebenfalls nicht dargestellten) Y-Stück verbindbar, das die Verbindung zum Patienten über ein Mundstück herstellt. Sowohl der erste als auch der zweite Inspirationsschlauch 3 bzw. 5 ist mit einer elektrischen Schlauchheizung ausgestattet, die als Heizwendel in die Schlauchwand integriert ist. In Fig. 1 ist lediglich die pneumatische Verbindung zwischen erstem und zweitem Inspirationsschlauch 3, 5 und dem Flüssigkeitsbehälter 11 über die Anschlussstücke 7, 9 dargestellt, da die elektrische Verbindung sämtlicher in Schläuchen verlaufender elektrischen Verbindungen über Kontaktelemente erfolgt, die in die Anschlussstücke 7, 9 integriert sind und den elektrischen Kontakt zu entsprechenden Gegenstück der Kontaktelemente an dem Gehäuse des Atemluftbefeuchters herstellen. Ein Exspirationsschlauch 13 ist über ein Anschlussteil 15 mit dem Beatmungsgerät verbindbar. Das andere Ende des Exspirationsschlauchs 13 wird in Betrieb mit dem Y-Stück nahe am Patienten verbunden.

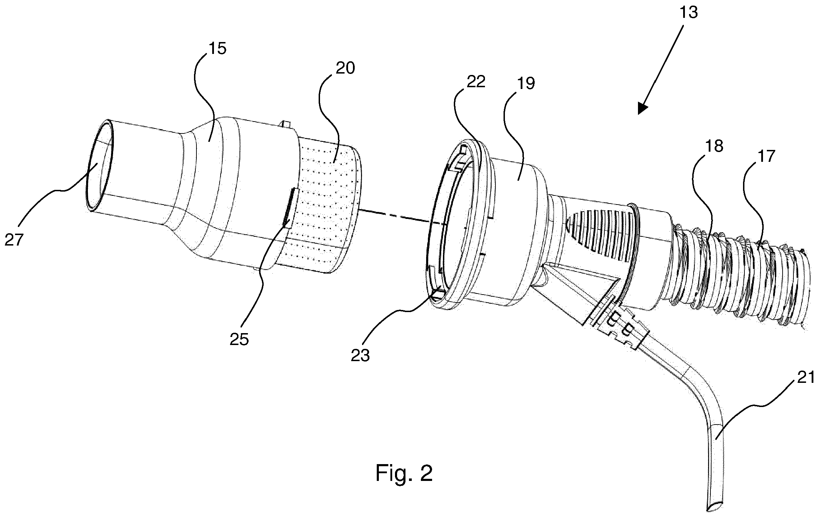

Der Exspirationsschlauch 13 stellt in der hier gezeigten Ausführungsform den erfindungsgemäßen Beatmungsschlauch dar und weist einen Schlauchabschnitt 17 auf, der mit einer in die Schlauchwand integrierten, als Heizwendel ausgebildeten elektrischen Heizvorrichtung 19 versehen ist. Zwischen Anschlussteil 15 und Schlauchabschnitt 17 weist der Exspirationsschlauch 13 ein Koppelteil 19 mit Filterelement 20 auf, wobei das Koppelteil 19 fest mit dem Schlauchabschnitt 17 und lösbar mit dem Anschlussteil 15 verbunden ist. Die Einzelheiten des Exspirationsschlauchs sind unter Bezugnahme auf Fig. 2 beschrieben.

Ein Verbindungselement 21 ist in der Fig. 2 dargestellten Ausführungsform zwischen dem Anschlussstück 2 des ersten Inspirationsschlauchs 3 und dem Koppelteil 19 des Exspirationsschlauchs 13 zur elektrischen Verbindung angeordnet. Das Anschlussstück 2 des ersten Inspirationsschlauchs 3 weist neben der pneumatischen Öffnung ein Hülsenelement auf, in das ein entsprechendes Steckelement des Verbindungselements 21 unlösbar eingesteckt ist. In gleicher Weise weist das Koppelteil 19 des Exspirationsschlauches 13 ein Hülsenelement auf, in die das entsprechende Steckelement des Verbindungselements 21 unlösbar eingesteckt ist. Der Begriff "unlösbar" ist in diesem Zusammenhang so zu verstehen, dass es für Bedienpersonal nicht ohne Weiteres möglich ist, die elektrischen Verbindungen zwischen Verbindungselement 21 und dem Anschlussstück 2 bzw. dem Koppelteil 19 zu lösen.

Da die Darstellung in Fig. 1 die Elemente betrifft, die üblicher Weise als medizinische Einmal-/Wegwerfartikel das notwendige austauschbare Zubehör für die Funktion eines Atemluftbefeuchters zusammen mit dem Beatmungsschlauchsystem bilden, sind Elemente wie das Y-Stück oder das Gehäuse des Atemluftbefeuchters an dieser Stelle weggelassen.

Das Material für die Schläuche, d. h. für den ersten Inspirationsschlauch 3, den zweiten Inspirationsschlauch 5 und den Exspirationsschlauch 13 ist aus einem geeigneten Kunststoffmaterial wie z. B. Polyethylen oder Polypropylen gebildet. Andere geeignete Materialien sind ebenfalls möglich. Die Schläuche werden in bekannter Technik extrudiert bzw. koextrudiert. Der Innendurchmesser der Schläuche beträgt üblicher Weise etwa 19 mm oder etwa 22 mm bei einem Beatmungssystem für Erwachsene, es können auch geringere Durchmesser wie 12 mm oder 15 mm verwendet werden, beispielsweise für Beatmungssysteme in Kinder- oder Säuglingsintensivstationen. Die Anschlussstücke, die den Übergang zwischen den Schläuchen der entsprechenden Geräte bzw. dem Y-Stück bilden, sind ebenfalls aus Kunststoffmaterial extrudiert. Da im medizinischen Bereich an die Materialien hohe Anforderungen gestellt werden, müssen die Materialen der ISO-Norm 5367-2000 genügen. Wie bereits erwähnt ist das erfindungsgemäße Beatmungsschlauchsystem entweder als medizinischer Einmal-/Wegwerfartikel ausgebildet oder alternativ als wieder verwendbarer medizinischer Artikel, der mittels Waschen und Autoklavieren wieder in einen neu verwendbaren Zustand versetzt werden kann. Alle Bestandteile des Beatmungsschlauchsystems müssen auch derart ausgebildet sein, dass sie beispielsweise keine Schadstoffe enthalten und einem kalten Desinfektionsmittel wie z.B. CIDEX, Sekusept, Korsolex usw. widerstehen.

Fig. 2 zeigt in perspektivischer Darstellung die Einzelheiten des Exspirationsschlauchs 13 der bevorzugten Ausführungsform des Beatmungsschlauches der vorliegenden Erfindung. Der Schlauchabschnitt 17 weist eine als Heizwendel 18 ausgebildete elektrische Heizvorrichtung auf, die in die Schlauchwand integriert ist. Das zylinderförmige Koppelteil 19 ist mit dem Schlauchabschnitt 17 verbunden, wobei die Heizwendel 18 elektrisch mit der in das Koppelteil 19 von außen nicht sichtbar integrierten elektrischen Heizvorrichtung in Verbindung steht. Führt die Heizwendel 18 des Schlauchabschnitts 17 einen Heizstrom, so fließt dieser auch in der Heizvorrichtung des Koppelteils 19. Alternativ ist es denkbar, die Heizvorrichtung des Koppelteils 19 separat elektrisch zu verbinden und beispielsweise über das Verbindungselement 21 zu steuern, das seitlich am Koppelteil 19 über ein Hülsenelement angeordnet ist. In der in Fig. 2 dargestellten Ausführungsform weist das Koppelteil 19 einen Flansch 22 auf, der auf seiner Innenseite eine Mehrzahl von Verschlusselementen 23 umfasst. Das Koppelteil 19 weist einen zylinderförmigen Hohlraum für die Aufnahme des Filterelements 20 auf.

Mit dem Koppelteil 19 verbindbar ist das Anschlussteil 15, das auf der Koppelseite zylinderförmig mit im Wesentlichen demselben Querschnittsmaß wie das Koppelteil 19 aufweist. Auf der Mantelfläche des Anschlussteils 15 sind außen als Vorsprünge ausgebildete Verschlusselemente 25 angeordnet, die dazu geeignet sind, in die Verschlusselemente 23 des Koppelteils 19 nach Art eines Bajonettverschlusses einzugreifen. Alternativ können auch andere lösbare Verbindungsmechanismen verwendet werden, z. B. Schraubverschluss, Magnetverschluss, Steckverschluss oder andere dem Fachmann geläufige Verbindungsarten.

Das Anschlussteil 15 geht vom Abschnitt größeren Querschnitts, der mit dem Koppelteil 19 verbindbar ist, trichterartig in einen zylinderförmigen Abschnitt 27 kleineren Querschnitts über, der zur Verbindung mit einem entsprechend ausgebildeten Gegenstück eines Beatmungsgerät geeignet ist. Das Anschlussteil 15 weist ebenfalls einen zylinderförmigen Hohlraum auf, in dem das Filterelement 20 aufgenommen ist. Zur Fixierung des Filterelements 20 innerhalb des Hohlraums können Aufnahmeelemente vorgesehen sein, die jedoch den Gasgemischfluss in Betrieb, d. h. bei bestimmungsgemäßer Verbindung von Anschlussteil 15 mit Koppelteil 19 nicht vollständig verhindern dürfen. Mit anderen Worten, die Befestigung des Filterelements 20 zwischen Koppelteil 19 und Anschlussteil 15 muss sowohl die ausreichende Wärmeübertragung von der Heizvorrichtung des Koppelteils 19 als auch den ausreichenden Gasgemischfluss durch den Beatmungsschlauch sicherstellen.

Deshalb sind innerhalb von Koppelteil 19 und Anschlussteil 15 verschiedene Konstruktionen möglich, die von der in Fig. 2 dargestellten Ausführungsform abweichen, jedoch die oben genannte Funktion erfüllen.

Es ist ebenfalls denkbar, dass auch das Anschlussteil 15 mit einer Heizvorrichtung versehen ist, die geeignet ist, das Filterelement 20 aufzuheizen. Dies kann beispielsweise durch eine in die Mantelfläche des Anschlussteils 15 integrierte elektrische Heizwendel erfolgen, die über die Verschlusselemente, in der hier dargestellten Ausführungsform über die Elemente des Bajonettverschlusses, elektrisch mit der Heizvorrichtung verbindbar ist. Dadurch entsteht eine elektrische Verbindung zwischen der Heizvorrichtung des Koppelteils 19 mit der Heizvorrichtung des Anschlussteils 15. Es sind auch andere Arten von Heizvorrichtungen möglich.

Weiterhin ist es möglich, dass das Verbindungselement 21 nicht am Koppelteil 19, sondern am Anschlussteil 15 angeordnet ist. Die elektrische Verbindung zwischen den beiden Heizvorrichtungen kann dann alternativ über das Verbindungselement 21 gespeist werden.

Mit dem Gegenstand der vorliegenden Erfindung wurde ein Beatmungsschlauch bereit gestellt, der die Filterung des Ausatemgases wirksam, zuverlässig und dauerhaft ermöglicht, die Kondensation von Wasser im Filter verhindert und die Anzahl zusätzlichen Elementen wie Schläuchen, elektrischen Leitungen sowie deren Anschlüsse und dergleichen minimiert.

## Patentansprüche

1. Beatmungsschlauch (13) für das Durchleiten eines Gasgemisches in einem Beatmungssystem mit
einem Schlauchabschnitt (17),
einem mit dem Schlauchabschnitt (17) fest verbundenen Koppelteil (19),
einem Anschlussteil (15), das an einen Schlauch oder ein Gerät anschließbar ist,
wobei zwischen dem Koppelteil (19) und dem Anschlussteil (15) ein Filterelement (20) zur Filterung des Gasgemisches angeordnet ist, und
einer ersten elektrischen Heizvorrichtung, die das Filterelement (20) umschließt und dadurch geeignet ist, das Filterelement (20) wirksam aufzuheizen, wobei das Koppelteil (19) lösbar mit dem Anschlussteil (15) verbunden ist und die erste elektrische Heizvorrichtung in das Koppelteil (19) integriert ist.

2. Beatmungsschlauch (13) nach Anspruch 1, **dadurch gekennzeichnet, dass** das Filterelement (20) und das Anschlussteil (15) integral ausgebildet sind.

3. Beatmungsschlauch (13) nach einem der vorhergehenden Ansprüche, **dadurch gekennzeichnet, dass** der Schlauchabschnitt (17), das Koppelteil (19) und das Anschlussteil (15) einen kreisförmigen Querschnitt aufweisen.

4. Beatmungsschlauch (13) nach einem der vorhergehenden Ansprüche, **dadurch gekennzeichnet, dass** die Verbindung zwischen Koppelteil (19) und Anschlussteil (15) lösbar als Schraub-, Steck- oder Bajonettverschluss ausgebildet ist.

5. Beatmungsschlauch (13) nach einem der vorhergehenden Ansprüche, **dadurch gekennzeichnet, dass** er als medizinischer Einmal-/Wegwerfartikel ausgebildet ist.

6. Beatmungsschlauch (13) nach einem der vorhergehenden Ansprüche, **dadurch gekennzeichnet, dass** der Schlauchabschnitt (17) eine zweite elektrische Heizvorrichtung (18) aufweist, die mit der ersten elektrischen Heizvorrichtung verbindbar ist.

7. Beatmungsschlauch (13) nach einem der vorhergehenden Ansprüche, **dadurch gekennzeichnet, dass** die erste und/oder die zweite elektrische Heizvorrichtung zumindest abschnittsweise als Heizwendel ausgebildet sind.

8. Beatmungsschlauch (13) nach Anspruch 6 oder 7, **dadurch gekennzeichnet, dass** die zweite elektrische Heizvorrichtung in die Wand des Schlauchabschnitts (17) integriert ist.

9. Beatmungsschlauch (13) nach einem der vorhergehenden Ansprüche, **dadurch gekennzeichnet, dass** der Schlauchabschnitt (17) und das Koppelteil (19) integral ausgebildet sind.

10. Beatmungsschlauch (13) nach einem der vorhergehenden Ansprüche, **dadurch gekennzeichnet, dass** die Anordnung von Koppelteil (19) und Filterelement (20) eine maximale Wärmeübertragung von der ersten elektrischen Heizvorrichtung auf das Filterelement (20) ermöglicht.

11. Beatmungsschlauch (13) nach Anspruch 10, **dadurch gekennzeichnet, dass** das Filterelement (20) zylinderförmig ausgebildet ist, wobei der Abstand zwischen äußerer Mantelfläche des Filterelements (20) und innerer Mantelfläche des Koppelteils (19) ungefähr 0,1 mm bis etwa 8 mm beträgt.

12. Beatmungsschlauch (13) nach einem der vorhergehenden Ansprüche, **dadurch gekennzeichnet, dass** er einen Sensor zur Erfassung von Parametern der Verbindung zwischen Koppelteil (19) und Anschlussteil (15) aufweist.

13. Beatmungsschlauch (13) nach einem der vorhergehenden Ansprüche, **dadurch gekennzeichnet, dass** das Anschlussteil (15) eine weitere elektrische Heizvorrichtung aufweist, die elektrisch mit der Heizvorrichtung des Koppelteils (19) verbindbar ist.

14. Beatmungsschlauchsystem mit einem Inspirationsschlauch (3, 5) und einem Exspirationsschlauch (13), der als Beatmungsschlauch nach einem der vorhergehenden Ansprüche ausgebildet ist.

## Claims

1. Respiratory tube (13) for passing through a gas mixture in a respiratory system, with a tube section (17), a coupling part (19) fixedly connected to the tube section (17), a connector part (15) which can be attached to a tube or an apparatus, wherein a filter element (20) is arranged between the coupling part (19) and the connector part (15) for filtering the gas mixture, and with a first electric heating device which surrounds the filter element (20) and is thereby suitable for efficiently heating up the filter element (20)
wherein the coupling part (19) is detachably connected to the connector part (15) and the first electric heating device is integrated into the coupling part (19).

2. Respiratory tube (13) according to claim 1 **characterised in that** the filter element (20) and the connector part (15) are of an integral design.

3. Respiratory tube (13) according to one of the preceding claims **characterised in that** the tube section (17), the coupling part (19) and the connector part (15) have a circular cross-section.

4. Respiratory tube (13) according to one of the preceding claims **characterised in that** the connection between the coupling part (19) and the connector part (15) is configured releasable as a screw, push-fit or bayonet closure.

5. Respiratory tube (13) according to one of the preceding claims **characterised in that** it is designed as a medical single-use/disposable article.

6. Respiratory tube (13) according to one of the preceding claims **characterised in that** the tube section (17) has a second electric heating device (18) which can be connected to the first electric heating device.

7. Respiratory tube (13) according to one of the preceding claims **characterised in that** the first and/or the second electric heating device is/are configured at least in certain sections as a heating coil.

8. Respiratory tube (13) according to claim 6 or 7 **characterised in that** the second electric heating device is integrated into the wall of the tube section (17).

9. Respiratory tube (13) according to one of the preceding claims **characterised in that** the tube section (17) and the coupling part (19) are of an integral design.

10. Respiratory tube (13) according to one of the preceding claims **characterised in that** the arrangement of the coupling part (19) and the filter element (20) enables maximum heat transfer from the first electric heating device to the filter element (20).

11. Respiratory tube (13) according to claim 10 **characterised in that** the filter element (20) has a cylindrical configuration wherein the distance between the outer lateral face of the filter element (20) and the inner lateral face of the coupling part (19) amounts to approximately 0.1 mm to about 8 mm.

12. Respiratory tube (13) according to one of the preceding claims **characterised in that** it has a sensor for detecting parameters of the connection between the coupling part (19) and connector part (15).

13. Respiratory tube (13) according to one of the preceding claims **characterised in that** the connector part (15) has a further electric heating device which can be connected electrically to the heating device of the coupling part (19).

14. Respiratory tube system with an inspiration tube (3, 5) and an expiration tube (13) which is configured as the respiratory tube according to one of the preceding claims.

## Revendications

1. Tuyau de ventilation respiratoire (13), destiné à faire passer un mélange gazeux dans un système de ventilation respiratoire, le tuyau comprenant :
un segment de tuyau (17),
une pièce d'accouplement (19) reliée solidement au segment de tuyau (17),
une pièce de raccordement (15), qui peut être raccordée à un tuyau ou à un appareil,
un élément filtrant (20) disposé entre la pièce d'accouplement (19) et la pièce de raccordement (15) afin de filtrer le mélange gazeux, et
un premier dispositif chauffant électrique, qui entoure l'élément filtrant (20) et qui est adapté à chauffer efficacement l'élément filtrant (20),
la pièce d'accouplement (19) étant reliée de manière détachable à la pièce de raccordement (15) et le premier dispositif chauffant électrique étant intégré dans la pièce d'accouplement (19).

2. Tuyau de ventilation respiratoire (13) selon la revendication 1, **caractérisé en ce que** l'élément filtrant (20) et la pièce de raccordement (15) sont formés d'une seule pièce.

3. Tuyau de ventilation respiratoire (13) selon l'une quelconque des revendications précédentes, **caractérisé en ce que** la section de tuyau (17), la pièce d'accouplement (19) et la pièce de raccordement (15) présentent une section transversale circulaire.

4. Tuyau de ventilation respiratoire (13) selon l'une quelconque des revendications précédentes, **caractérisé en ce que** la liaison entre la pièce d'accouplement (19) et la pièce de raccordement (15) est réalisée de manière détachable sous la forme d'une fermeture à vis, à encliquetage ou à baïonnette.

5. Tuyau de ventilation respiratoire (13) selon l'une quelconque des revendications précédentes, **caractérisé en ce qu'**il est réalisé sous la forme d'un article médical à usage unique/jetable.

6. Tuyau de ventilation respiratoire (13) selon l'une quelconque des revendications précédentes, **caractérisé en ce que** la section de tuyau (17) comprend un dispositif chauffant électrique (18), qui peut être relié au premier dispositif chauffant électrique.

7. Tuyau de ventilation respiratoire (13) selon l'une quelconque des revendications précédentes, **caractérisé en ce que** le premier et/ou le deuxième dispositif électrique chauffant sont réalisés au moins en partie sous la forme de filaments chauffants.

8. Tuyau de ventilation respiratoire (13) selon la revendication 6 ou 7, **caractérisé en ce que** le deuxième dispositif chauffant électrique est intégré dans la paroi de la section de tuyau (17).

9. Tuyau de ventilation respiratoire (13) selon l'une quelconque des revendications précédentes, **caractérisé en ce que** la section de tuyau (17) et la pièce d'accouplement (19) sont réalisées d'un seul tenant.

10. Tuyau de ventilation respiratoire (13) selon l'une quelconque des revendications précédentes, **caractérisé en ce que** l'agencement de la pièce d'accouplement (19) et de l'élément filtrant (20) permet un transfert de chaleur maximal du premier dispositif chauffant électrique à l'élément filtrant (20).

11. Tuyau de ventilation respiratoire (13) selon la revendication 10, **caractérisé en ce que** l'élément filtrant (20) est cylindrique, la distance entre la surface extérieure de l'élément filtrant (20) et la surface intérieure de la pièce d'accouplement (19) atteignant approximativement 0,1 mm à environ 8 mm.

12. Tuyau de ventilation respiratoire (13) selon l'une quelconque des revendications précédentes, **caractérisé en ce qu'**il comprend un capteur permettant de détecter des paramètres de la liaison entre la pièce d'accouplement (19) et la pièce de raccordement (15).

13. Tuyau de ventilation respiratoire (13) selon l'une quelconque des revendications précédentes, **caractérisé en ce que** la pièce de raccordement (15) comprend un autre dispositif chauffant électrique qui peut être relié électriquement au dispositif chauffant de la pièce d'accouplement (19).

14. Système de tuyau de ventilation respiratoire comprenant un tuyau d'inspiration (3, 5) et un tuyau d'expiration (13), qui est réalisé sous la forme d'un tuyau de ventilation respiratoire selon l'une quelconque des revendications précédentes.
